# EUROPEAN PATENT APPLICATION

(11) **EP 4 260 871 A1**
(43) Date of publication of application: **18.10.2023**
(21) Application number: 21903209.1
(22) Date of filing: 26.11.2021
(51) Int. Cl.: A61K 39/395, A61P 27/06, C07K 16/28, A61K 9/08, A61K 9/10

(54) **OPTICAL NERVE PROTECTING AGENT CONTAINING ANTI-LRP1 ANTIBODY**

(30) Priority: 08.12.2020 JP 2020203477
(71) Applicant: Tokyo University of Pharmacy & Life Sciences, Hachioji-shi, Tokyo 192-0392 (JP); National Institutes of Biomedical Innovation, Health and Nutrition, Ibaraki-shi, Osaka 567-0085 (JP)
(72) Inventor: HAYASHI Hideki, Hachioji-shi, Tokyo 192-0392 (JP); TAKAGI Norio, Hachioji-shi, Tokyo 192-0392 (JP); KIKKAWA Yamato, Hachioji-shi, Tokyo 192-0392 (JP); KAMADA Haruhiko, Ibaraki-shi, Osaka 567-0085 (JP); NAGATA Satoshi, Ibaraki-shi, Osaka 567-0085 (JP)
(74) Representative: D Young & Co LLP
(86) International application number: PCT/JP2021/043471
(87) International publication number: WO 2022/124105

(57) **Abstract**

The present invention relates to an optic nerve protecting agent containing an anti-low density lipoprotein receptor-related protein 1 (LRP1) antibody or an anti-LRP1 antibody fragment.

## Description

### Technical Field

The present invention relates to an optic nerve protecting agent containing an anti-LRP1 antibody.

### Background Art

Glaucoma is a progressive neurodegenerative disease in which retinal ganglion cells constituting an optic nerve degenerate for a certain reason and visual field defects occur, and is the first leading cause of blindness in Japan and the second leading cause of blindness in the world.

Currently, the only approach for progression prevention and treatment of glaucoma is lowering intraocular pressure. In Japan, patients with normal intraocular pressure account for 70% of those with glaucoma, and there are lots of cases where symptoms of glaucoma still progress even if the intraocular pressure is controlled by an intraocular pressure lowering agent. Therefore, development of a glaucoma treatment method based on a new mechanism of action has been desired.

In glaucoma, retinal ganglion cells constituting the optic nerve are damaged. For the purpose of elucidating the mechanism of optic nerve degeneration in optic nerve diseases, the inventors of the present invention have focused on a lipoprotein derived from glial cells, being a factor that protect these retinal ganglion cells, and studied suppression of retinal nerve cell death in optic nerve degeneration such as glaucoma. As a result, it was confirmed in an *in vitro* system that an apolipoprotein E-containing lipoprotein (E-LP) derived from glial cells has a neuroprotective effect against apoptosis (Non Patent Literature 1, Non Patent Literature 2, and Non Patent Literature 3). Here, the apolipoprotein E-containing lipoprotein (E-LP) is an apolipoprotein-binding lipoprotein in which apolipoprotein E, being a kind of apolipoprotein, and a lipid are chemically associated with each other.

Furthermore, the inventors of the present invention have found that when E-LP is injected into the vitreous body of a mouse in which a large amount of lipoproteins are present, E-LP has a neuroprotective effect against apoptosis even in an *in vivo* system, and further, a complex of E-LP and a neuroprotective molecule such as low density lipoprotein receptor-related protein-1 (LRP1) that is a receptor of E-LP similarly has a neuroprotective effect against apoptosis in the *in vivo* system (Patent Literature 1).

In other words, the inventors of the present invention have so far clarified that E-LP provides protection of rat primary cultured retinal ganglion cells from apoptosis via LRP1 that is a receptor of E-LP, and have found that E-LP can be used as an inhibitor of a neuroprotective inhibitory effect (Patent Literature 1) .

### Prior-arts

### Non Patent Literature Documents

Non Patent Literature Document 1: Hayashi, H., et al., J. Neurosci., February 21, 2007, 27(8): 1933-1941
Non Patent Literature Document 2: Hayashi, Hideki, et al.: Neurochemistry Vol. 49 No.2/3 Page 483(2010)
Non Patent Literature Document 3: Hayashi, H., et al., J Biol Chem: Vol. 284 No.43 pp. 29605-29613 (2009)

### Patent Literature Document

Patent Literature Document 1: JP 6031718

### Summary of the Invention

### Problem to be Solved by the Invention

An object of the present invention is to provide an optic nerve protecting agent containing an anti-LRP1 antibody.

### Means to Solve the Problem

The inventors of the present invention have further conducted intensive studies, and elucidated that an antibody against LRP1, being a receptor of E-LP, suppresses nerve disorders induced in primary cultured retinal ganglion cells and a rat glaucoma model. This result suggests a possibility of glaucoma treatment with an antibody based on the protective effect on the retinal ganglion cells via LRP1.

Namely, the present invention relates to:
[1] an optic nerve protecting agent for protecting optic nerve in a subject, comprising an anti-low density lipoprotein receptor-related protein 1 (LRP1) antibody or an anti-LRP1 antibody fragment;
[2] the optic nerve protecting agent according to [1], wherein the anti-LRP1 antibody or the antibody fragment specifically binds to C2 domain of the LRP1;
[3] the optic nerve protecting agent according to [1] or [2], wherein the anti-LRP1 antibody or the antibody fragment recognizes an epitope:
   (a) in a region of amino acids at positions 786 to 1165 in an amino acid sequence of LRP1 having an amino acid sequence represented by SEQ ID NO: 1,
   (b) in a region of amino acids at positions 787 to 1166 in an amino acid sequence of LRP1 having an amino acid sequence represented by SEQ ID NO: 2,
   (c) in a region of amino acids at positions 786 to 1165 in an amino acid sequence of LRP1 having an amino acid sequence represented by SEQ ID NO: 3, or
   (d) in a region of amino acids at positions 787 to 1166 in an amino acid sequence of LRP1 having an amino acid sequence represented by SEQ ID NO: 4;
[4] the optic nerve protecting agent according to any one of [1] to [3], wherein
   (i) the anti-LRP1 antibody or the antibody fragment comprises:
      a heavy chain complementarity determining region 1 (VH CDR1) that comprises an amino acid sequence having a sequence identity of 80% or more to an amino acid sequence represented by SEQ ID NO: 9;
      a heavy chain complementarity determining region 2 (VH CDR2) that comprises an amino acid sequence having a sequence identity of 80% or more to an amino acid sequence represented by SEQ ID NO: 10;
      a heavy chain complementarity determining region 3 (VH CDR3) that comprises an amino acid sequence having a sequence identity of 80% or more to an amino acid sequence represented by SEQ ID NO: 11;
      a light chain complementarity determining region 1 (VL CDR1) that comprises an amino acid sequence having a sequence identity of 80% or more to an amino acid sequence represented by SEQ ID NO: 12;
      a light chain complementarity determining region 2 (VL CDR2) that comprises an amino acid sequence having a sequence identity of 80% or more to an amino acid sequence represented by SEQ ID NO: 13; and
      a light chain complementarity determining region 3 (VL CDR3) that comprises an amino acid sequence having a sequence identity of 80% or more to an amino acid sequence represented by SEQ ID NO: 14,
      or
   (ii) the anti-LRP1 antibody or the antibody fragment comprises:
      a heavy chain variable region that comprises an amino acid sequence having a sequence identity of 80% or more to an amino acid sequence represented by SEQ ID NO: 15, and
      a light chain variable region that comprises an amino acid sequence having a sequence identity of 80% or more to an amino acid sequence represented by SEQ ID NO: 16,
      or
   (iii) the anti-LRP1 antibody or the antibody fragment comprises an amino acid sequence having a sequence identity of 80% or more to an amino acid sequence represented by SEQ ID NO: 19;
[5] the optic nerve protecting agent according to any one of [1] to [4], wherein the anti-LRP1 antibody or the antibody fragment is a humanized antibody or a human antibody;
[6] the optic nerve protecting agent according to any one of [1] to [5], wherein the anti-LRP1 antibody or the antibody fragment binds to LRP1 with a dissociation constant (Kd) of 1 × 10⁻⁷ M to 1 × 10⁻¹¹ M;
[7] a composition comprising the optic nerve protecting agent according to any one of [1] to [6];
[8] the composition according to [7], wherein the composition is topically administered to the subject;
[9] the composition according to [8], wherein the topical administration is intravitreal administration;
[10] the composition according to [8] or [9], wherein the composition is in a form of eye drops;
[11] the composition according to any one of [8] to [10], for treating glaucoma;
[12] the composition according to any one of [8] to [11], wherein the subject has an intraocular pressure of 10 to 21 mmHg;
[13] an anti-LRP1 antibody or an anti-LRP1 antibody fragment, comprising:
   (i) a heavy chain complementarity determining region 1 (VH CDR1) that comprises an amino acid sequence having a sequence identity of 80% or more to an amino acid sequence represented by SEQ ID NO: 9;
      a heavy chain complementarity determining region 2 (VH CDR2) that comprises an amino acid sequence having a sequence identity of 80% or more to an amino acid sequence represented by SEQ ID NO: 10;
      a heavy chain complementarity determining region 3 (VH CDR3) that comprises an amino acid sequence having a sequence identity of 80% or more to an amino acid sequence represented by SEQ ID NO: 11;
      a light chain complementarity determining region 1 (VL CDR1) that comprises an amino acid sequence having a sequence identity of 80% or more to an amino acid sequence represented by SEQ ID NO: 12;
      a light chain complementarity determining region 2 (VL CDR2) that comprises an amino acid sequence having a sequence identity of 80% or more to an amino acid sequence represented by SEQ ID NO: 13; and
      a light chain complementarity determining region 3 (VL CDR3) that comprises an amino acid sequence having a sequence identity of 80% or more to an amino acid sequence represented by SEQ ID NO: 14,
      or
   (ii) a heavy chain variable region that comprises an amino acid sequence having a sequence identity of 80% or more to an amino acid sequence represented by SEQ ID NO: 15, and
      a light chain variable region that comprises an amino acid sequence having a sequence identity of 80% or more to an amino acid sequence represented by SEQ ID NO: 16,
      or
   (iii) an amino acid sequence having a sequence identity of 80% or more to an amino acid sequence represented by SEQ ID NO: 19; and
[14] a composition comprising the anti-LRP1 antibody or the anti-LRP1 antibody fragment according to [13].

### Effects of Invention

According to the present invention, an optic nerve protecting agent containing an anti-LRP1 antibody can be provided.

### Brief Description of the Drawings

Fig. 1 shows an outline of a neuroprotective mechanism via an antibody.
Fig. 2A shows a schematic diagram of LRP1 and each ligand-binding domain protein.
Fig. 2B shows a diagram plotting reactivity of each clone (in a horizontal axis).
Fig. 2C shows amino acid sequences of a heavy chain and a light chain of scFv.
Fig. 2D shows SDS-PAGE of each purified scFv.
Fig. 2E shows results of ELISA for each purified scFv.
Fig. 3A shows a protective effect on retinal ganglion cells of scFv (Group #1) against glutamate neurotoxicity.
Fig. 3B shows a protective effect on retinal ganglion cells of scFv (Group #2) against glutamate neurotoxicity.
Fig. 3C shows a protective effect on retinal ganglion cells of scFv (Group #3) against glutamate neurotoxicity.
Fig. 4 shows a protective effect on retinal ganglion cells of scFv (C2-D11) on rats with NMDA-induced optic nerve degeneration (analysis results of immunoblotting of a retina).
Fig. 5 shows a protective effect on retinal ganglion cells of scFv (C2-D11) on rats with NMDA-induced optic nerve degeneration (summary of the analysis results of immunoblotting of the retina).
Fig. 6 shows a protective effect on retinal ganglion cells of scFv (C2-D11) on rats with NMDA-induced optic nerve degeneration (HE staining of retinal tissue sections).
Fig. 7 shows a protective effect on retinal ganglion cells of scFv (C2-D11) on rats with NMDA-induced optic nerve degeneration (analysis of HE stained images of the retinal tissue sections and the number of retinal ganglion cells).
Fig. 8A shows a protective effect on retinal ganglion cells of scFv (C2-D11) on rats with NMDA-induced optic nerve degeneration (analysis of the HE stained images of the retinal tissue sections and thickness of an inner plexiform layer).
Fig. 8B shows a protective effect on retinal ganglion cells of scFv (C2-D11) on rats with NMDA-induced optic nerve degeneration (analysis of the HE stained images of the retinal tissue sections and thickness of the inner plexiform layer) obtained from a test commissioned to a third party organization.
Fig. 9 shows thickness of the retinal inside from the inner plexiform layer measured by an optical coherence tomography.
Fig. 10 shows a schematic diagram illustrating a method for producing an anti-LRP1 human IgG1 antibody from scFv.
Fig. 11A shows results of ELISA for an anti-LRPl human IgGl antibody.
Fig. 11B shows results of ELISA for an anti-OVA antibody (as a control).
Fig. 12 shows binding of the anti-LRPl human IgGl antibody to immobilized LRP1-Fc.
Fig. 13 shows a protective effect on retinal ganglion cells using intracellular calcium influx by glutamate stimulation as an index.
Fig. 14 shows localization of the anti-LRP1 human IgG1 antibody after intravitreal administration.
Fig. 15 shows neuroprotective effects by the anti-LRP1 human IgG1 antibody at various concentrations.
Fig. 16A shows analysis results of electrophoresis gel staining of LRP1 recombinant protein-deficient variants.
Fig. 16B shows analysis results of immunoblotting of the LRP1 recombinant protein-deficient variants.

### Detailed Description of Embodiments

Hereinafter, the present invention will be described in more detail.

Specifically, a first aspect of the present invention relates to an optic nerve protecting agent containing an anti-LRP1 antibody or an anti-LRP1 antibody fragment.

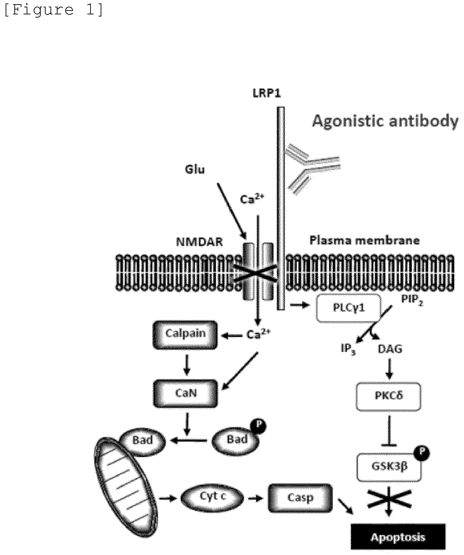

As described above, the inventors of the present invention have so far found that E-LP protects rat primary cultured retinal ganglion cells from apoptosis via LRP1, being one of receptors of E-LP, and E-LP can be used as an inhibitor of a neuroprotective inhibitory effect. In the present invention, the anti-LRP1 antibody is used in place of E-LP to protect ganglion cells from apoptosis. Specifically, as shown in Fig. 1, the anti-LRP1 antibody specifically binds to LRP1 present in a membrane, thereby activating a neuroprotective molecule, and inactivating or suppressing a neurodegeneration inducing molecule, and thus avoids apoptosis of ganglion cells and protects nerves. Here, examples of the neuroprotective molecule include phospholipase C ("PLCγ1" in Fig. 1) and protein kinase Cδ ("PKCδ" in Fig. 1), and examples of the neurodegeneration inducing molecule include NMDA-type glutamate receptor ("NMDAR" in Fig. 1), calcium ("Ca^{2 +}" in Fig. 1), and GSK3β.

The inventors of the present invention have further developed the above studies and succeeded in inducing the protection of optic nerve cells via LRP1 with the anti-LRP1 antibody or the anti-LRP1 antibody fragment. When E-LP is used, there is a concern that the antibody may bind to a target other than LRP1, but the antibody is specific to the target, and has excellent uniformity and high stability. From this point of view, the antibody according to the present invention can also be useful in pharmaceutical applications.

LRP1 also includes a protein containing an amino acid sequence in which one or more amino acids are deleted, substituted, inserted and/or added in the amino acid sequence of the protein, and being functionally equivalent to the protein. Here, the "functionally equivalent protein" is a protein having an activity equivalent to the activity of the protein, and the "functionally equivalent protein" includes a protein having, for example, a sequence identity of 80% or more, 83% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.5% or more, 99% or more, 99.1% or more, 99.2% or more, 99.3% or more, 99.4% or more, 99.5% or more, 99.6% or more, 99.7% or more, 99.8% or more, or 99.9% or more to the amino acid sequence of the protein.

Therefore, the term "LRP1" includes a protein having an amino acid sequence having a sequence identity of 80% or more, 83% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.5% or more, 99% or more, 99.1% or more, 99.2% or more, 99.3% or more, 99.4% or more, 99.5% or more, 99.6% or more, 99.7% or more, 99.8% or more, or 99.9% or more to the amino acid sequence represented by a sequence number specified below, and having equivalent activity.

LRP1 includes an amino acid sequence represented by any of SEQ ID NO: 1 to SEQ ID NO: 4. Here, the amino acid sequence of LRP1 can be obtained from a database known in the art. Examples of the amino acid sequence of LRP1 are shown below:
- SEQ ID NO: 1 (Low-density lipoprotein receptor-related protein 1 preproprotein [Homo sapiens], NCBI Reference Sequence: NP_002323.2 (4544 amino acids)),
- SEQ ID NO: 2 (Low-density lipoprotein receptor-related protein 1 [Mus musculus], NCBI Reference Sequence: NP_032538.2 (4545 amino acids)),
- SEQ ID NO: 3 (Low-density lipoprotein receptor-related protein 1 [Macaca mulatta], NCBI Reference Sequence: XP_015007567.1 (4544 amino acids))
- SEQ ID NO: 4 (Low-density lipoprotein receptor-related protein 1 [Rattus norvegicus], NCBI Reference Sequence: NP_001123962.1 (4545 amino acids))

The LRP1 in the present invention includes a protein having an amino acid sequence having, for example, a sequence identity of 80% or more, 83% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.5% or more, 99% or more, 99.1% or more, 99.2% or more, 99.3% or more, 99.4% or more, 99.5% or more, 99.6% or more, 99.7% or more, 99.8% or more, or 99.9% to the amino acid sequence specified by SEQ ID NO: 1, 2, 3, or 4.

Further, examples of a base sequence of a gene encoding LRP1 are shown below:
- SEQ ID NO: 5 (Homo sapiens LDL receptor related protein 1 (LRP1), mRNA, NCBI Reference Sequence: NM_002332.3 (14923 base pairs)),
- SEQ ID NO: 6 (Mus musculus low density lipoprotein receptor-related protein 1 (Lrp1), mRNA, NCBI Reference Sequence: NM_008512.2 (14907 base pairs))
- SEQ ID NO: 7 (PREDICTED: Macaca mulatta LDL receptor related protein 1 (LRP1), mRNA, NCBI Reference Sequence: XM_015152081.2 (14900 base pairs))
- SEQ ID NO: 8 (Rattus norvegicus LDL receptor related protein 1 (Lrp1), mRNA, NCBI Reference Sequence: NM_001130490.1 (14714 base pairs)).

As used herein, the term "antibody" is intended to include not only an antibody itself, but also a fragment of the antibody, particularly an antigen-binding fragment. Therefore, as used herein, in a case where the term "antibody" is described, the term "antibody" is intended to include an antibody and an antibody fragment thereof. Specifically, the term "anti-LRP1 antibody" is intended to include both an anti-LRP1 antibody and an anti-LRP1 antibody fragment.

The term "antibody fragment" includes, for example, a single-chain antibody (scFv), a scFv dimer, a disulfide-stabilized V-region fragment (dsFv), Fv, Fab, Fab', F(ab')2, a domain antibody, and the like.

In one embodiment, the anti-LRP1 antibody may be a single-chain antibody (scFv). In another embodiment, the anti-LRP1 antibody may be an IgG antibody, an IgA antibody, an IgE antibody, or an IgM antibody.

In a case where the anti-LRP1 antibody is a single-chain antibody (scFv), it can be produced by a method known in the art, and for example, it may be produced by chemical synthesis in addition to being produced by a microorganism using a phagemid, a plasmid, and the like. For example, the anti-LRP1 antibody, being a single-chain antibody, is formed by linking a heavy chain variable domain and a light chain variable domain by a flexible linker.

In a case where the anti-LRP1 antibody is an IgG antibody, the antibody may be a mouse antibody, a chimeric antibody, a humanized antibody or a human antibody, preferably a humanized antibody or a human antibody. In a case where the antibody is an IgG antibody, it can be produced by a method known in the art. One example of the method for producing an IgG antibody will be described later, but the method is not limited thereto.

The optic nerve protecting agent containing an anti-LRP1 antibody or an anti-LRP1 antibody fragment according to the present invention protects the optic nerve *in vitro* or *in vivo.* Here, the term "optic nerve protecting agent" includes not only a case of containing other components in addition to the anti-LRP1 antibody and/or the antigen fragment thereof, but also a case of containing only the anti-LRP1 antibody and/or the antigen fragment thereof.

"Protection of optic nerve" refers to preventing degeneration of optic nerve cells, and specifically refers to inhibiting degeneration of retinal ganglion cells and reducing disorders in retinal ganglion cells, and protecting axon of retinal ganglion cells constituting the optic nerve. Here, the term "degeneration" with respect to nerves includes apoptosis, necrosis and necroptosis. The "protection of the optic nerve" can be confirmed by means known in the art, and can be confirmed, for example, by a method of hematoxylin-eosin staining (hereinafter, it is also referred to as "HE staining") of retinal sections, immunoblotting of a marker protein of retinal ganglion cells, or electroretinogram (hereinafter, it is also referred to as "ERG"). Further, since degeneration of nerves can be detected by an immunohistological method, the "protection of optic nerve" may be confirmed using this method.

In one embodiment, the optic nerve protecting agent protects the optic nerve in a subject. As used herein, the term "subject" broadly includes animals. The animal is, for example, a vertebrate, preferably a mammal. As used herein, the term "mammal" is used to refer to any animal classified as a mammal, including, but not limited to, a human, mouse, rat, monkey, cow, horse, sheep, dog, and cat. As used herein, a preferred mammal is a human.

The anti-LRP1 antibody or the antibody fragment thereof is an antibody that specifically binds to the C2 domain of LRP1.

In one embodiment, the anti-LRP1 antibody or the antibody fragment thereof recognizes an epitope:
(a) in a region of amino acids at positions 786 to 1165 in the amino acid sequence of LRP1 having an amino acid sequence represented by SEQ ID NO: 1,
(b) in a region of amino acids at positions 787 to 1166 in the amino acid sequence of LRP1 having an amino acid sequence represented by SEQ ID NO: 2, or
(c) in a region of amino acids at positions 786 to 1165 in the amino acid sequence of LRP1 having an amino acid sequence represented by SEQ ID NO: 3,
(d) in a region of amino acids at positions 787 to 1166 in the amino acid sequence of LRP1 having an amino acid sequence represented by SEQ ID NO: 4.

In the present invention, an "epitope" means a partial peptide of an LRP1 polypeptide having antigenicity and/or immunogen in the body of an animal, preferably a mammal, more preferably a human, mouse, rat or monkey. An epitope that is the partial peptide of LRP1 having antigenicity can be determined by a method known to those skilled in the art, such as immunoassay. The epitope can be determined, for example, by the following method. To start with, various partial structures of the LRP1 polypeptide are produced using known oligopeptide synthesis techniques. Specifically, it can be determined by examining a series of polypeptides sequentially shortened by an arbitrary length from the C-terminus or N-terminus of the LRP1 polypeptide, determining a rough recognition site, then synthesizing shorter peptides, and examining the reactivity with them. As another specific method, it can be determined by expressing each domain structure as a recombinant protein using each domain structure of LRP1 as a basic unit, and examining a difference in binding to these recombinant proteins.

In a case where the anti-LRP1 antibody or the antibody fragment thereof is an anti-LRP1 human IgG antibody, the anti-LRP1 antibody or the antibody fragment thereof binds to LRP1 with a dissociation constant (Kd) of, for example, 1 × 10⁻⁵ M to 1 × 10⁻¹² M, preferably 1 × 10⁻⁷ M to 1 × 10⁻¹¹ M, more preferably 1 × 10⁻⁸ M to 1 × 10⁻¹⁰ M, and still more preferably 1 × 10⁻⁸ M to 1 × 10⁻⁹ M.

The anti-LRP1 antibody or the antibody fragment thereof according to the present invention includes, for example, the following complementarity determining regions (CDRs):
a heavy chain complementarity determining region 1 (VH CDR1) that includes an amino acid sequence having a sequence identity of 80% or more, 83% or more, 85% or more, 90% or more, 93% or more, 95% or more, 97% or more, 98% or more, or 99% or more to an amino acid sequence represented by SEQ ID NO: 9;
a heavy chain complementarity determining region 2 (VH CDR2) that includes an amino acid sequence having a sequence identity of 80% or more, 83% or more, 85% or more, 90% or more, 93% or more, 95% or more, 97% or more, 98% or more, or 99% or more to an amino acid sequence represented by SEQ ID NO: 10;
a heavy chain complementarity determining region 3 (VH CDR3) that includes an amino acid sequence having a sequence identity of 80% or more, 83% or more, 85% or more, 90% or more, 93% or more, 95% or more, 97% or more, 98% or more, or 99% or more to an amino acid sequence represented by SEQ ID NO: 11;
a light chain complementarity determining region 1 (VL CDR1) that includes an amino acid sequence having a sequence identity of 80% or more, 83% or more, 85% or more, 90% or more, 93% or more, 95% or more, 97% or more, 98% or more, or 99% or more to an amino acid sequence represented by SEQ ID NO: 12;
a light chain complementarity determining region 2 (VL CDR2) that includes an amino acid sequence having a sequence identity of 80% or more, 83% or more, 85% or more, 90% or more, 93% or more, 95% or more, 97% or more, 98% or more, or 99% or more to an amino acid sequence represented by SEQ ID NO: 13; and
a light chain complementarity determining region 3 (VL CDR3) that includes an amino acid sequence having a sequence identity of 80% or more, 83% or more, 85% or more, 90% or more, 93% or more, 95% or more, 97% or more, 98% or more, or 99% or more to an amino acid sequence represented by SEQ ID NO: 14.

In a preferred embodiment, the anti-LRP1 antibody or the antibody fragment thereof includes:
a heavy chain complementarity determining region 1 (VH CDR1) that includes the amino acid sequence represented by SEQ ID NO: 9;
a heavy chain complementarity determining region 2 (VH CDR2) that includes the amino acid sequence represented by SEQ ID NO: 10;
a heavy chain complementarity determining region 3 (VH CDR3) that includes the amino acid sequence represented by SEQ ID NO: 11;
a light chain complementarity determining region 1 (VL CDR1) that includes the amino acid sequence represented by SEQ ID NO: 12;
a light chain complementarity determining region 2 (VL CDR2) that includes the amino acid sequence represented by SEQ ID NO: 13; and
a light chain complementarity determining region 3 (VL CDR3) that includes the amino acid sequence represented by SEQ ID NO: 14.

**[Table 1]**

| | |
|---|---|
| Heavy chain complementarity determining region 1 (VH CDR1) | Light chain complementarity determining region 1 (VL CDR1) |
| SY AMS (SEQ ID NO: 9) | QGDSLHSYYAS (SEQ ID NO: 12) |
| Heavy chain complementarity determining region 2 (VH CDR2) | Light chain complementarity determining region 2 (VL CDR2) |
| AISGSGGSTYYADSVKG (SEQ ID NO: 10) | GKNNRPS (SEQ ID NO: 13) |
| Heavy chain complementarity determining region 3 (VH CDR3) | Light chain complementarity determining region 3 (VL CDR3) |
| RTGTLFDY (SEQ ID NO: 11) | NSSQAQRNAVV (SEQ ID NO: 14) |

In another embodiment, the anti-LRP1 antibody or the antibody fragment thereof includes:
- a heavy chain variable region that includes an amino acid sequence having a sequence identity of 80% or more, 83% or more, 85% or more, 90% or more, 93% or more, 95% or more, 97% or more, 98% or more, or 99% or more to an amino acid sequence represented by SEQ ID NO: 15, and
- a light chain variable region that includes an amino acid sequence having a sequence identity of 80% or more, 83% or more, 85% or more, 90% or more, 93% or more, 95% or more, 97% or more, 98% or more, or 99% or more to an amino acid sequence represented by SEQ ID NO: 16.

In another preferred embodiment, the anti-LRP1 antibody or the antibody fragment thereof includes:
- a heavy chain variable region that includes the amino acid sequence represented by SEQ ID NO: 15, and
- a light chain variable region that includes the amino acid sequence represented by SEQ ID NO: 16. The heavy chain variable region and the light chain variable region are preferably linked by a linker.

**[Table 2]**

| Amino acid sequence of heavy chain variable region |
|---|
| EVQLLESGGGLVQPGGSLRLSCAASGFTFSSYAMSWVRQAPGKGLEWVSAISGSGGSTYYADSVKGRF TISRDNSKNTLYLQMNSLRAEDTAVYYCAKRTGTLFDYWGQGTLVTVSS (SEQ ID NO: 15) |

| Amino acid sequence of light chain variable region |
|---|
| SSELTQDPAVSVALGQTVRITCQGDSLRSYYASWYQQKPGQAPVLVIYGKNNRPSGIPDRFSGSSSGNTA SLTITGAQAEDEADYYCNSSQAQRNAVVFGGGTKLTVLG (SEQ ID NO: 16) |

| Base sequence of heavy chain variable region |
|---|
| gaagtccaactgctggaatcgggtggtggtctggtgcaaccgggcggctcgctgcgtctgtcatgtgctgcgtcgggctttacctttagctcttatgcaatg tcctgggtgcgtcaggcaccgggtaaaggtctggaatgggttagcgcaattagtggctccggcggtagcacctattacgccgattctgttaaaggtcgttt taccatctcacgcgacaactcgaaaaatacgctgtatctgcagatgaacagtctgcgcgcagaagataccgctgtctattactgcgcaaaacggactgg gactttgttcgactactggggccaaggtacgctggttacggttagcagc (SEQ ID NO: 17) |

| Base sequence of light chain variable region |
|---|
| tcgtctgaactgacgcaagatccggctgtttctgtggcactgggtcaaacggttcgtatcacgtgtcaaggcgattcactgcgctcctattacgcaagttgg tatcagcaaaaaccgggccaggctccggtgctggttatttacggcaaaaacaatcgtccgtccggtatcccggatcgcttttcaggcagctctagtggtaa caccgcgtcgctgaccattacgggtgcgcaagccgaagatgaagcagactattactgcaatagctcccaggcgcagcggaatgcggttgtctttggtggt ggtacgaaactgaccgtcctgggc (SEQ ID NO: 18) |

In still another embodiment, the anti-LRP1 antibody or the antibody fragment thereof includes an amino acid sequence having a sequence identity of 80% or more, 83% or more, 85% or more, 90% or more, 93% or more, 95% or more, 97% or more, 98% or more, or 99% or more to the amino acid sequence represented by SEQ ID NO: 19.

In still another preferred embodiment, the anti-LRP1 antibody or the antibody fragment thereof includes the amino acid sequence represented by SEQ ID NO: 19.

**[Table 3]**

| Amino acid sequence of scFv antibody |
|---|
| EVQLLESGGGLVQPGGSLRLSCAASGFTFSSYAMSWVRQAPGKGLEWVSAISGSGGSTYYADSVKGRF TISRDNSKNTLYLQMNSLRAEDTAVYYCAKRTGTLFDYWGQGTLVTVSSGGGGSGGGGSGGGGSSELT QDPAVSVALGQTVRITCQGDSLRSYYASWYQQKPGQAPVLVIYGKNNRPSGIPDRFSGSSSGNTASLTIT GAQAEDEADYYCNSSQAQRNAVVFGGGTKLTVLG (SEQ ID NO: 19) |

| Base sequence of scFv antibody |
|---|
| gaagtccaactgctggaatcgggtggtggtctggtgcaaccgggcggctcgctgcgtctgtcatgtgctgcgtcgggctttacctttagctcttatgcaatg tcctgggtgcgtcaggcaccgggtaaaggtctggaatgggttagcgcaattagtggctccggcggtagcacctattacgccgattctgttaaaggtcgttt taccatctcacgcgacaactcgaaaaatacgctgtatctgcagatgaacagtctgcgcgcagaagataccgctgtctattactgcgcaaaacggactgg gactttgttcgactactggggccaaggtacgctggttacggttagcagcggcggtggtggcagcggtggtggcggctcgggtggtggcggctcgtctgaa ctgacgcaagatccggctgtttctgtggcactgggtcaaacggttcgtatcacgtgtcaaggcgattcactgcgctcctattacgcaagttggtatcagca aaaaccgggccaggctccggtgctggttatttacggcaaaaacaatcgtccgtccggtatcccggatcgcttttcaggcagctctagtggtaacaccgcgt cgctgaccattacgggtgcgcaagccgaagatgaagcagactattactgcaatagctcccaggcgcagcggaatgcggttgtctttggtggtggtacga aactgaccgtcctgggc (SEQ ID NO: 20) |

Another embodiment of the present invention relates to an expression vector for producing an anti-LRP1 antibody or an anti-LRP1 antibody fragment. The expression vector includes one or more nucleic acids encoding the anti-LRP1 antibody of the present invention or a part thereof. Here, the nucleic acid is operably linked to a control sequence recognized by a host cell in a case where the host cell is transfected with a vector.

The expression vector according to the present invention includes, for example, at least one nucleic acid selected from the followings:
- a DNA encoding a protein having the amino acid sequence represented by SEQ ID NO: 15 and a DNA encoding a protein having the amino acid sequence represented by SEQ ID NO: 16,
- a DNA having a polynucleotide sequence represented by SEQ ID NO: 17 and a DNA having a polynucleotide sequence represented by SEQ ID NO: 18,
- a DNA hybridizing with a DNA having a polynucleotide sequence complementary to the polynucleotide sequence represented by SEQ ID NO: 17 and the polynucleotide sequence represented by SEQ ID NO: 18 under a stringent condition, and encoding a protein that specifically binds to LRP1,
- a DNA including a polynucleotide sequence having a sequence identity of 80% or more to the polynucleotide sequence represented by SEQ ID NO: 17 and a polynucleotide sequence having a sequence identity of 80% or more to the polynucleotide sequence represented by SEQ ID NO: 18, and encoding a protein that specifically binds to LRP1,
- a DNA including a degenerate isomer of the polynucleotide sequence represented by SEQ ID NO: 17 and a degenerate isomer of the polynucleotide sequence represented by SEQ ID NO: 18,
- a DNA encoding a protein having the amino acid sequence represented by SEQ ID NO: 19,
- a DNA having a polynucleotide sequence represented by SEQ ID NO: 20,
- a DNA hybridizing with a DNA having a polynucleotide sequence complementary to the polynucleotide sequence represented by SEQ ID NO: 20 under a stringent condition, and encoding a protein that specifically binds to LRP1,
- a DNA including a polynucleotide sequence having a sequence identity of 80% or more to the polynucleotide sequence represented by SEQ ID NO: 20, and encoding a protein that specifically binds to LRP1,
- a DNA encoding a protein including an amino acid sequence having 1 to 10 amino acids deleted, substituted, inserted and/or added to the amino acid sequence of the protein encoded by the polynucleotide sequence represented by SEQ ID NO: 20, and encoding a protein that specifically binds to LRP1,
- a DNA including a degenerate isomer of the polynucleotide sequence represented by SEQ ID NO: 20.

The present invention also provides a host cell including one or more expression vectors, and a method for producing an anti-LRP1 antibody or an antigen fragment thereof using such a host cell. Here, the production method includes at least one of culturing a host cell containing an expression vector that produces an anti-LRP1 antibody or an antigen fragment thereof in a medium, and isolating the anti-LRP1 antibody or the antigen fragment thereof from the host cell or the medium. The present invention also provides a host cell genetically modified to express an anti-LRP1 antibody or an antigen fragment thereof in the absence of a vector as described above.

Yet another aspect of the invention is a composition containing the anti-LRP1 antibody or the anti-LRP1 antibody fragment or an optic nerve protecting agent previously described. Here, the term "composition" is intended to include not only a case of containing an anti-LRP1 antibody, an antigen fragment thereof, and other components other than an optic nerve protecting agent, but also a case of containing only an anti-LRP1 antibody, an antigen fragment thereof, and/or an optic nerve protecting agent (in other words, the case where other components are not contained). In one embodiment, the composition according to the present invention is a composition containing an anti-LRP1 antibody or an anti-LRP1 antibody fragment and a solvent. The composition according to the present invention is preferably a pharmaceutical composition containing an anti-LRP1 antibody or an anti-LRP1 antibody fragment at a therapeutically effective amount.

In one embodiment, the composition according to the present invention is a composition for topical administration, which is administered topically to a subject. Topical administration is, for example, instillation administration, subconjunctival injection, intravitreal administration and intraocular implant.

The composition according to the present invention can be administered orally or parenterally, preferably parenterally. Examples of a dosage form of the composition include a tablet, a capsule, a fine granule, a pill, a troche, a transfusion, an injection, eye drops, a suppository, an ointment, a patch and the like.

In one embodiment, the composition according to the present invention is a pharmaceutical composition for prevention or treatment of glaucoma. As described above, a certain percentage of glaucoma patients are patients with normal intraocular pressure, and there are many cases which still progresses even when the intraocular pressure is controlled by an intraocular pressure lowering agent. Therefore, the pharmaceutical composition of the present invention is preferably used for glaucoma patients having normal intraocular pressure. Specifically, glaucoma patients have an intraocular pressure, for example, in the range of 10 to 23 mmHg, preferably 10 to 21 mmHg, more preferably in the range of 10 to 20 mmHg.

Examples of glaucoma include primary open-angle glaucoma, normal-tension glaucoma, aqueous excessive glaucoma, ocular hypertension, acute angle-closure glaucoma, chronic angle-closure glaucoma, plateau iris syndrome, combined mechanism glaucoma, steroid-induced glaucoma, capsular glaucoma of crystalline lens, pigmentary glaucoma, amyloid glaucoma, neovascular glaucoma, malignant glaucoma, and the like.

As used herein, with respect to glaucoma, the term "treatment" is intended to include inhibiting or reducing glaucoma in addition to treating glaucoma. The term "treatment" specifically includes inhibiting a progression of optic nerve degeneration, and preventing apoptosis, necrosis and/or necroptosis of the optic nerve.

The subject includes a subject suffering from glaucoma who has received treatment by administration of an intraocular pressure lowering agent in the past, in particular, a subject suffering from glaucoma who has received treatment by administration of an intraocular pressure lowering agent in the past, and whose glaucoma symptoms are still progressing. Here, the intraocular pressure lowering agent is an intraocular pressure lowering agent known in the art, and examples thereof include an adrenaline α2 receptor agonist (Aiphagan (registered trademark) ophthalmic solution), a prostaglandin F2α derivative (Tapros (registered trademark) ophthalmic solution), a Rho kinase inhibitor (Granatec (registered trademark) ophthalmic solution), and the like, but are not limited thereto. The subject may further include a subject that has undergone a surgical procedure for the treatment of glaucoma.

In a case where the composition according to the present invention is applied to a subject for the prophylaxis or treatment of glaucoma, the composition is administered parenterally, preferably topically, more preferably intravitreally. Thus, in a case where the composition according to the present invention is a pharmaceutical composition, it is for example for parenteral administration, preferably for topical administration, more preferably for vitreous administration.

The dosage form of the composition according to the present invention is not particularly limited, but when applied to a subject for treatment of glaucoma, it is preferably eye drops or an ophthalmic injection.

The composition according to the present invention may typically contain a pharmaceutically acceptable additive, for example, a pharmaceutically acceptable excipient, binder, auxiliary agent, lubricant, solvent, diluent, stabilizer, emulsifier, preservative, carrier, solubilizing agent, tonicity agent and the like known in the art.

A dosage of the composition according to the present invention may be appropriately adjusted depending on the age, weight, disease state, and the like of the subject.

The optic nerve protecting agent and the pharmaceutical composition according to the present invention may be used as a combination agent in combination with an additional agent or pharmaceutical composition. With regard to the combination agent, active ingredients in the respective pharmaceutical compositions (that is, the first active ingredient and the second active ingredient) may be administered together, sequentially or individually, in one combined unit dosage form or in two separate unit dosage forms. A therapeutically effective amount of each active ingredient of the combination according to the present invention may be administered simultaneously, individually or in any order, sequentially or successively. In the combination agent, the first active ingredient and the second active ingredient may be individually present in each of plural units. The combination agent also includes, for example, a kit containing respective active ingredients and instructions for use.

According to the present invention, an optic nerve protecting agent and a composition containing an anti-LRP1 antibody or an anti-LRP1 antibody fragment that specifically binds to LRP1 can be provided. Since an anti-LRP1 antibody or an anti-LRP1 antibody fragment having high specificity is used, it is possible to specifically act on a factor involved in degeneration of optic nerve cells, and therefore degeneration of the optic nerve can be suppressed while reducing undesired side effects.

Furthermore, according to the present invention, an anti-LRP1 antibody or an anti-LRP1 antibody fragment can protect the optic nerve with a mechanism of action other than intraocular pressure reduction, and thus is expected to be a new therapeutic approach to glaucoma. Furthermore, due to the mechanism of avoiding degeneration of the optic nerve cells, it is expected to be able to provide another option of treatment in patients with glaucoma who have received treatment with the intraocular pressure lowering agent in the past and patients with optic nerve degeneration with normal intraocular pressure.

### EXAMPLES

Hereinafter, the present invention will be described more specifically with reference to examples, but the present invention is not limited by these examples at all.

### <Primary culture of retinal ganglion cells>

The primary culture of retinal ganglion cells was performed using a two-day old Sprague Dawley (SD) rat according to a method slightly modified from the method of Barres et al. (Barres et al., 1988) (Hayashi et al., J. Biol. Chem. 2009). Isolated retinal ganglion cells (RGCs) were suspended in a basic medium of RGC medium (containing 1 mM glutamine, 5 ug/ml insulin, 60 ug/ml N-acetylcysteine, 62 ng/ml progesterone, 16 ug/ml putrescine, 40 ng/ml sodium selenite, 0.1 mg/ml bovine serum albumin, 40 ng/ml triiodothyronine, 0.1 mg/ml transferrin, 1 mM sodium pyruvate, 2% B27 supplement (Invitrogen, Carlsbad, CA), 10 µM forskolin (Sigma, St. Louis, MO), 50 ng/ml brain-derived neurotrophic factor (BDNF; PeproTech, Rocky Hill, NJ), 50 ng/ml ciliary neurotrophic factor (CNTF; PeproTech), and 50 ng/ml basic fibroblast growth factor (bFGF; PeproTech). A 96 well plate was coated with poly-d-lysine (Sigma) and laminin (Sigma), and mounted with RGCs to 5,000 cells per well for a 96 well plate and 5,000 cells per culture insert for a microdish. The cells were subsequently cultured for at least 10 days before experiments.

### <Preparation of reconstituted apolipoprotein E-containing lipoprotein>

The reconstituted apolipoprotein E-containing lipoprotein was prepared as described in the prior literature (Hayashi et al., J. Neurosci. 2007). This reconstituted lipoprotein E-containing lipoprotein was composed of 1-palmitoyl-2-oleyl-glycerophosphocholine (POPC; P3017, Sigma), cholesterol (C3045, Sigma) and recombinant human apolipoprotein E, and a molar ratio of the components was 100 : 10 : 1 or 100 : 0 : 1. A solution containing a preparation medium, plasma or reconstituted apolipoprotein E-containing lipoprotein was subjected to a noncontinuous sucrose gradient. A composition of the solution used was 3 ml for a density of 1.30 g/ml, 3 ml for a density of 1.2 g/ml, 3 ml for a density of 1.1 g/ml and 6 ml for a density of 1.006 g/ml. The sucrose gradient was subjected to centrifugation using an SRP28SA1 rotor (Hitachi, Tokyo, Japan) at 100,000 g for 72 hours at 4°C. Ten fractions (1.5 ml) were collected from the top of the gradient, and subjected to immunoblotting for apolipoprotein E as follows. Fractions containing apolipoprotein E (typically fractions 5 to 7) were combined, and concentrated using an Amicon Ultra filter (50 kDa molecular weight cut-off; Millipore, Bedford, MA). The mass of lipoprotein was adjusted by a cholesterol concentration (2 µg/ml) for HDL, and by a protein concentration (100 ng/ml) for reconstituted lipoprotein. The concentrations of the cholesterol and protein were measured by a LabAssay cholesterol kit (Wako) and a BCA protein assay kit (Thermo Fisher Scientific Inc., Rockford, IL), respectively.

### <Screening of anti-LRP1 antibody>

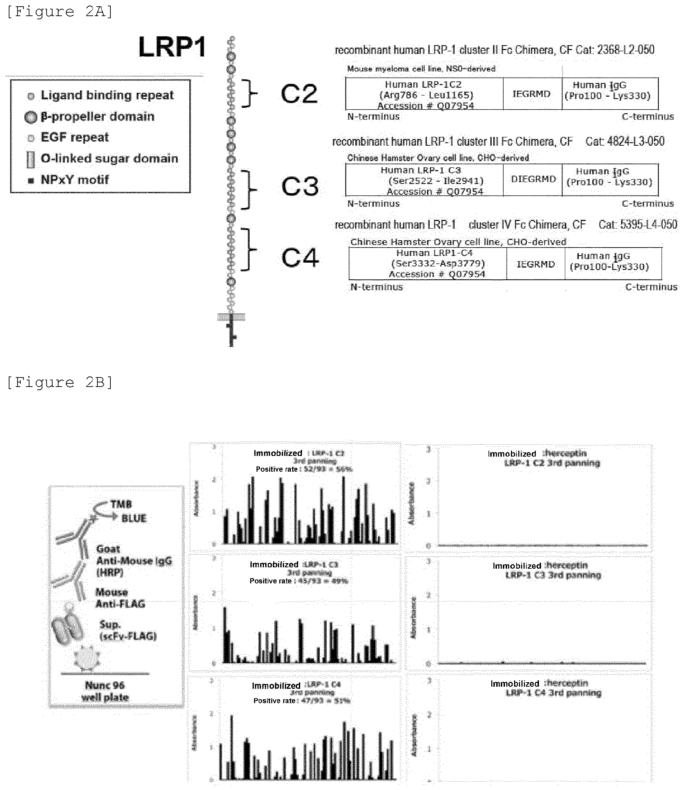

Using commercially available proteins derived from LRP1 partial domain and Human Fc (three species: C2, C3, C4) as an antigen, scFv for each cluster of LRP1 (that is, C2, C3 and C4) was obtained from a human scFv phage library. Fig. 2A shows a schematic diagram of the antigen used, i.e. LRP1.

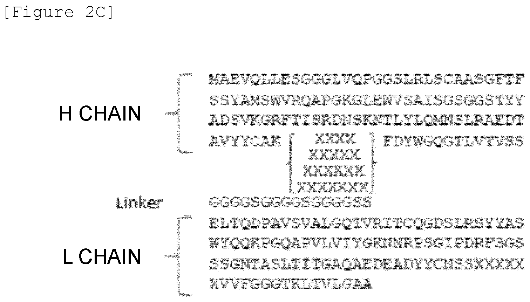

scFvs that specifically bind to clusters 2, 3, and 4 of LRP1 (that is, C2, C3 and C4) were subjected to screening (panning) from the human scFv phagelibrary. As a result, a large number of scFvs specific to each of the LRP1 clusters 2, 3, and 4 were obtained. The results of plotting the reactivity of each clone (in the horizontal axis) are shown in Fig. 2B. Multiple phage clones (52, 45 and 47 clones, respectively) that specifically react to each domain of LRPl (ligand binding clusters C2, C3 and C4) were obtained from the clones after three times of pannings (93 clones each). Each scFv was subjected to sequenceing, and most of them were confirmed to have different CDR3 sequences. The scFv library used for the screening was derived from DP47-DPL16 (PDB: 3GHE), which is a library in which random sequences were introduced into CDR3 regions of each of the heavy chain and the light chain. Fig. 2C shows the amino acid sequences of the heavy chain (in the drawing, "H chain") and the light chain (in the drawing, "L chain") of scFv, with randomized moieties shown as "X" in each chain.

### <Evaluation of binding site in LRP1 of anti-LRP1 antibody>

Four scFv clones for the LRP1 clusters were purified. These clones were selected for the stability of E. coli retention of phagemid and stability in purification as an index. The sequence of the VH and VL CDR3 of the four scFv clones, and a feed rate are shown in the following table.

**[Table 4]**

| Clone name | VH CDR3 | VL CDR3 | Feed rate (in PBS) |
|---|---|---|---|
| C2_D11 | RTGTLFDY | QAQRNA | 0.758 mg/ml x 100 ul |
| C2_F8 | TTGHLFDY | NPRRQR | 0.508 mg/ml x 100 ul |
| C2_G3 | TTGELFDY | RRRKHH | 0.739 mg/ml x 100 ul |
| C2_G9 | NTGASFDY | RYRKHP | 0.675 mg/ml x 100 ul |
| Anti-OVA (Control) | GFTFSSYAFDY | SLRSYY | 1.0 mg/ml x 100 ul |

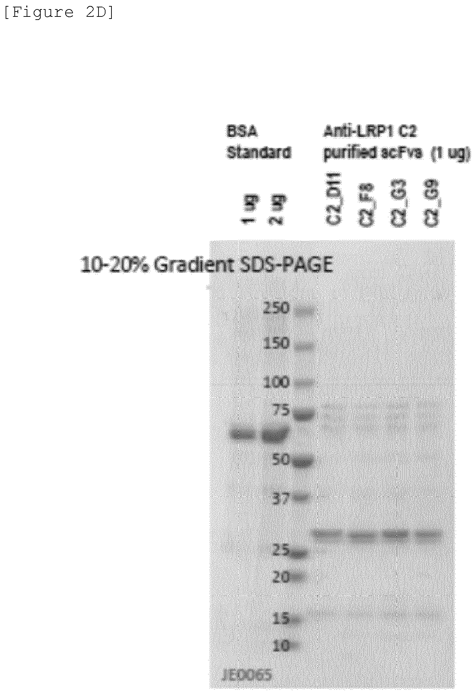

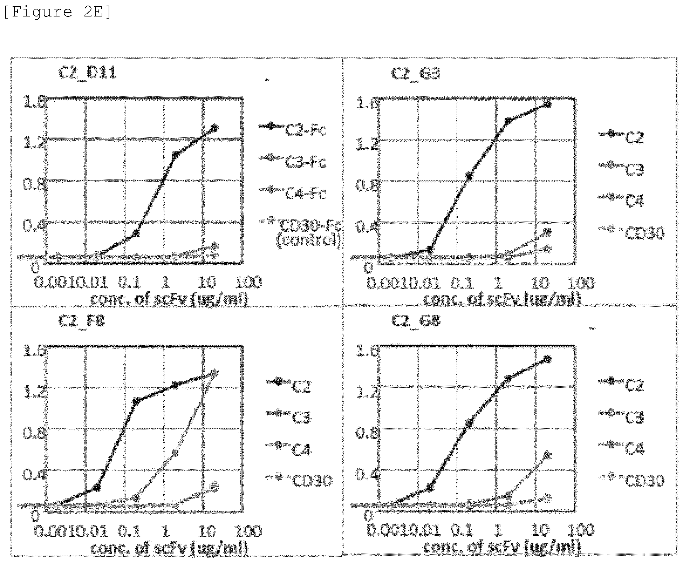

SDS-PAGE of each purified scFv is shown in Fig. 2D. Each purified scFv had a purity of 90% or more. Fig. 2E shows the results of ELISA for C2-D11, C2-F8, C2-G3, and C2-G9 among the purified scFvs.

Materials and conditions used in ELISA are shown in the following table.

### [Table 5]

**Table 5: Details of ELISA for binding test of antibody to antigen**

| | |
|---|---|
| ELISA to test mAbs for reactivity with antigen | <Samples or standards> |
| <Materials> | Prepare Antibody sample |
| - Nunc-Immuno^{™} Plate MaxSorp^{™} (Nunc 439454) | Wash the plate 1x with Wash buffer |
| - Plate seal | Add 50ul/well of the antibody solution |
| - PBS | Incubate at RT for >60 min or 4 degree overnight |
| - Wash buffer: PBS containing 0.05% Tween20 and 0.05% azide | 2nd Antibody |
| | Dilute ALP-labeled 2nd Ab (For example, goat anti-mouse Fc at 1:1000 with BBBT) |
| - BSA blocking buffer (BBB): PBS containing 0.5% BSA and 0,1% azide | |
| | Wash the plates 2x with Wash buffer |
| - BBBT: BRB containing 0.05% Tween20 | Add 50ul/well of the diluted 2nd antibody |
| . p-Nitrophenylphosphoric Acid Disodium Salt | |
| - Ix ALP buffer: 0.2 M diethanolamine, 0.5 mM Magnesium chloride, 0.04% azide, pH 9.8 | <Development> |
| | Prepare I mg/ml pnpp solution with 1x ALP buffer |
| | Wash the plate 4x with Wash buffer |
| <Coating> | Add 50 ul/well of the pnpp solution |
| Dilute antigen protein to 2 ug/ml in PBS. | Incubate at RT for 10-30 min (optional) Add 50ul/well of 0.5M EDTA to step the reaction |
| Add 50 ul/well (50-100 ng/well) to Nunc-Immuno^{™} | |
| Plate MaxSorp^{™} (Nunc 439454) | |
| Seal the plate, mix to make the solution surface even | Measure OD at 405nm |
| Incubate at RT for 4-6 hrs or at 4 degree for overnight | |
| <Blocking (optional, , usually not necessary)> | |
| Add 100ul/well blocking buffer (for example, 1% BSA in PBS, 0.1% sodium azide) | |
| | |
| Incubate at R.T. for >30 min | |

The results shown in Fig. 2E revealed that at a low concentration of 100 ng/ml, these scFvs show specific reactivity to C2-Fc (C2 domain).

### <Protective effect of scFv on glutamate-induced neurotoxicityin primary cultured retinal ganglion cells>

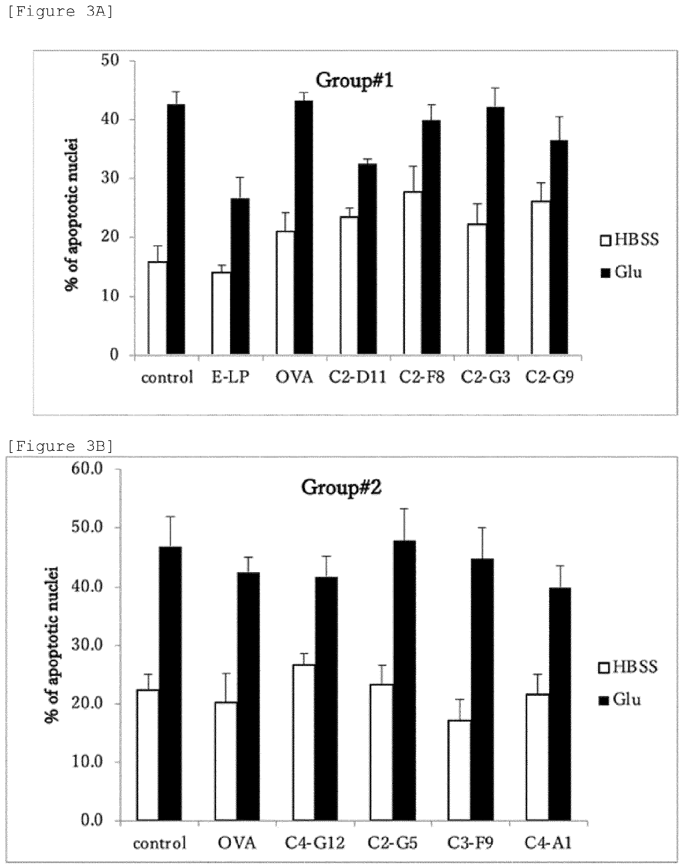

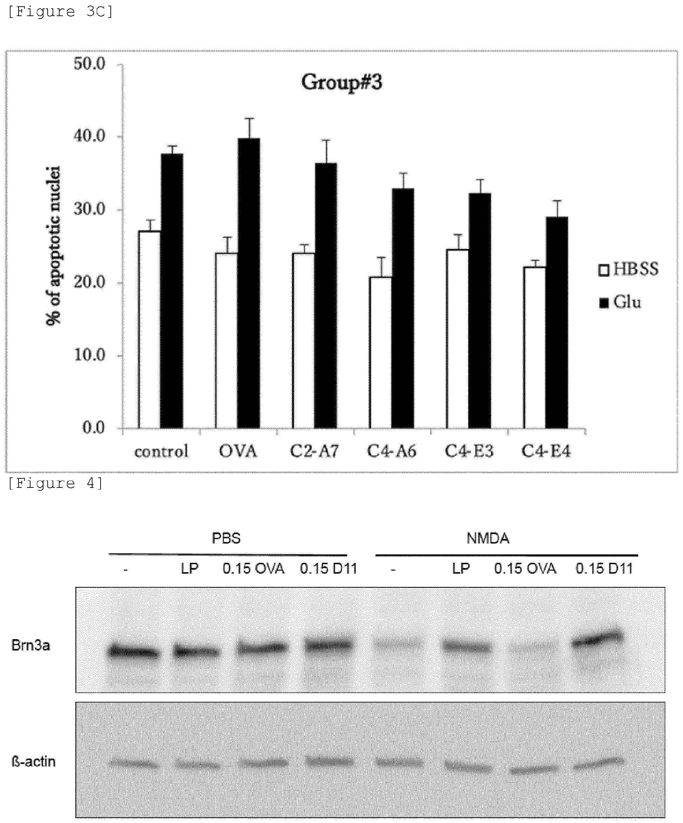

Apoptosis was induced with 300 µM glutamic acid and 10 µM glycine for two hours. scFv was used at a concentration of 1 µg/ml, and E-LP was used at a concentration of 100 ng/ml as a positive control. Anti-OVA (ovalbumin) scFv was used as a negative control. Nuclear aggregation 24 hours after glutamate-induced neurotoxicity was analyzed by Hoechst staining. The results regarding the protective effect on retinal ganglion cells of scFv in Group #1 (n = 4 to 5), Group #2 (n = 5) and Group #3 (n = 5) are shown in Figs. 3A, 3B and 3C, respectively. From these results, among the scFv clones binding to the Ligand-Binding Cluster C2 (D11, F8, G3, G9, G5, and A7), D11 was confirmed to have a protective effect on retinal ganglion cells, while no neuroprotective effect was observed in the scFv clone binding to C3 and the scFv clone binding to C4.

### <Immunoblotting>

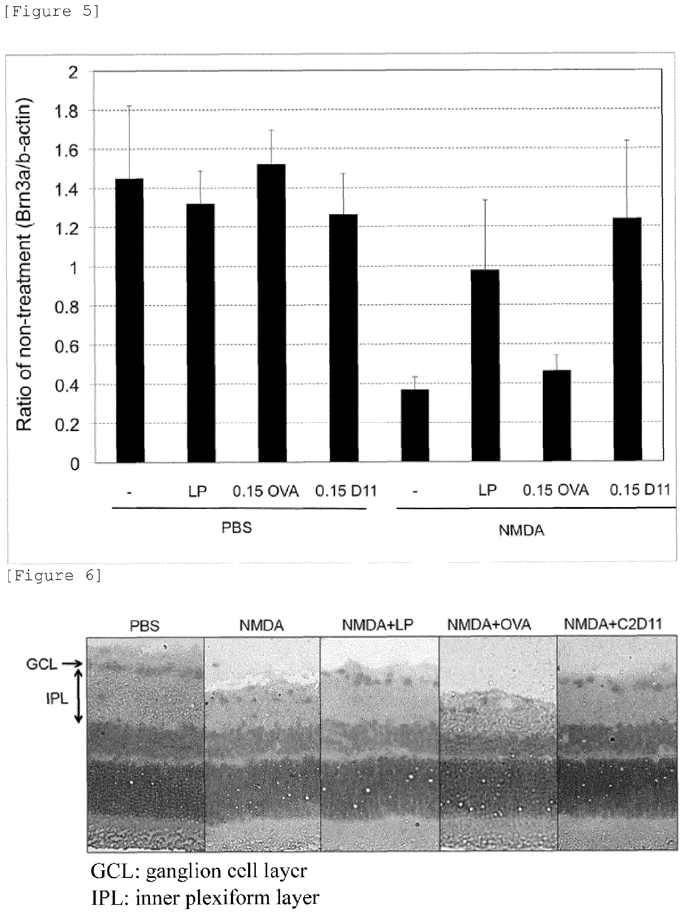

The proteins were dissolved in 62.5 mM Tris-HCl (pH 6.8), 10% glycerol, 2% sodium dodecyl sulfate (SDS) and 5% β-mercaptoethanol (sample buffer) and boiled for five minutes. The proteins were separated by electrophoresis on polyacrylamide gels containing 0.1% SDS, and then transferred to a polyvinylidene difluoride membrane. The membranes were incubated for one hour at room temperature with 5% skim milk in TBS-T (10 mM Tris-HCl (pH 7.4), 150 mM NaCl and 0.1% Tween 20), and then probed overnight at 4°C with a primary antibody in TBS-T containing 5% bovine serum albumin. The membranes were subsequently probed for one hour at room temperature with peroxidase-conjugated goat anti-mouse IgG (Thermo), goat anti-human IgG (Thermo), or mouse anti-goat IgG (Thermo). Immunoreactive proteins were visualized with enhanced chemiluminescence (GE Healthcare, Buckinghamshire, UK) or Super Signal West Dura (Thermo). The primary antibodies used were as follows: human C2-D11 IgG (0.5 µg/ml), mouse antibody β-actin (a5441, diluention 1 : 10,000, Sigma), goat anti-human Brn-3a (sc-31984, diluention 1 : 1000, Santa Cruz). Brn-3a is a marker protein of retinal ganglion cells, and β-actin was used as a loading control. scFv (C2-D11) was administered intravitreally simultaneously with N-methyl-D-aspartic acid (NMDA). The results are shown in Figs. 4 and 5.

Fig. 4 shows analysis results of immunoblotting of the retina, demonstrating the protective effect on retinal ganglion cells of scFv (C2-D11) on rats with NMDA-induced optic nerve degeneration. Fig. 5 shows the protective effect on retinal ganglion cells of scFv (C2-D11) on the rats with NMDA-induced optic nerve degeneration by summarizing the results of immunoblot analysis (3 to 6 cases) of Fig. 4 (summary of the analysis results of the immunoblotting of the retina). From Figs. 4 and 5, the protective effect on retinal ganglion cells of scFv (C2-D11) was confirmed.

### <Induction and detection of apoptosis in retinal ganglion cells (RGCs)>

RGCs were washed twice with Hanks' Balanced Salt Solution (HBSS; 14170, Invitrogen) containing 2.4 mM CaCl₂, 20 mM HEPES and no magnesium, and then incubated at 37°C for two hours with 300 µM glutamic acid and 10 µM glycine with or without E-LP, C2-D11 scFv or C2-D11 IgG in HBSS containing 2.4 mM CaCl₂, 20 mM HEPES and no magnesium. After glutamic acid treatment, RGCs were cultured at 37°C for 22 hours with a culture medium for RGC without forskolin, BDNF, CNTF or bFGF. For the detection of apoptosis, RGCs were stained with 1 ug/ml of Hoechst 33342 (346-07951, Kumamoto, DOJINDO). Fluorescence images were observed using an Olympus IX 71 microscope. Fragmented or shrunken nuclei stained with Hoechst dye were counted as apoptotic neurons, and round and smooth nuclei were counted as healthy neurons.

### <NMDA-induced retinal degeneration by C2-D11 antibody in rats>

Intravitreal injection of NMDA (Sigma) was performed with minor modifications, similar to the method described in the prior literature (Tsutsumi T. et al., Invest Ophthalmol Vis Sci. 2016, 57(14): 6461-6473). Specifically, 7-week-old rats were anesthetized with 5% isoflurane, and then maintained with 2.5% isoflurane. The pupil was dilated by instillation of phenylephrine hydrochloride and tropicamide. Subsequently, PBS as a vehicle control or 20 nmol/eye NMDA with or without 5 ng/eye E-LP, 0.15 ug/eye C2-D11 scFv or 0.75 ug/eye C2-D11 IgG in a total volume of 4 ul was injected into the vitreous cavity. Injections were performed under a microscope using a 34-gauge needle (Nanopass, Terumo, Tokyo, Japan) connected to a microsyringe (80008, Hamilton, Reno, NV) equipped with an injection pump (Fusion 200, Chemyx Inc., Stafford, TX), and the needle was inserted approximately 1.0 mm behind the corneal limbus. The eyeballs were enucleated three days after NMDA injection and retinae were removed from the sclerae, added into lysis buffer [1% Triton X-100; MP Biomedicals (Santa Ana, CA, CA, USA), 0.1% sodium deoxycholate; Wako (Osaka, Japan), 1% EDTA; DOJINDO (Kumamoto, Japan), Complete protease inhibitor cocktail; Roche (Basel, Switzerland), PhosStop phosphatase inhibitor cocktail; Roche, 50 mM Tris-buffered saline), and then sonicated with Ultrasonic Liquid Processor Q125 (QSONICA) at 4°C for 20 seconds (2 seconds sonication × 10 times). Protein concentration of retinal sample was measured by a BCA protein assay kit (Thermo Fisher Scientific Inc.) and subjected to immunoblotting.

### <Frozen specimen preparation and HE staining of rat eyeballs>

The frozen specimen preparation and HE staining of eyeballs were performed according to the following procedure.
(1) Eyeballs were removed on the third day after NMDA +/C2-D11 scFv administration.
(2) The eyeballs were gently grasped with ring tweezers, the conjunctiva was cut with scissors, and then the optic nerve was cut long and immersed in Super Fix at 4°C for two hours.
(3) After fixation for two hours, an eye cup was prepared.
(4) The procedure was returned to Super Fix again, and the eye cup was fixed for 22 hours (overnight, at 4°C).
(5) After 22 hours, the eye cup was transferred to 20% sucrose and soaked at room temperature until it submerged. Then the eye cup was transferred to 30% sucrose and repeated until 40% sucrose.
(6) The eye cup submerged in 40% sucrose was removed.
(7) The OCT compound was placed in Cryomold into which the eye cup was submerged.
(8) The eye cup was embedded in isopentane cooled with liquid nitrogen.
(9) The temperature of a cryostat sample stand was set to -20°C.
(10) The eye cup was thinly sliced at a thickness of 10 um.
(11) Thinly sliced sections were HE stained.
(12) After HE staining, the number of retinal ganglion cells (RGCs) and the thickness of the inner plexiform layer (IPL) at positions 1 mm to 1.5 mm from the optic disc were measured. The results are shown in Figs. 6, 7, and 8. In Fig. 6, GCL represents a ganglion cell layer, and IPL represents an inner plexiform layer.

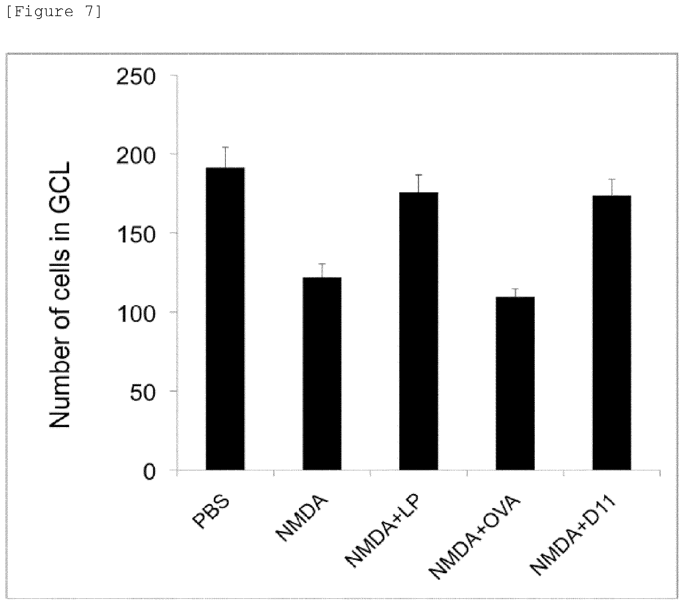

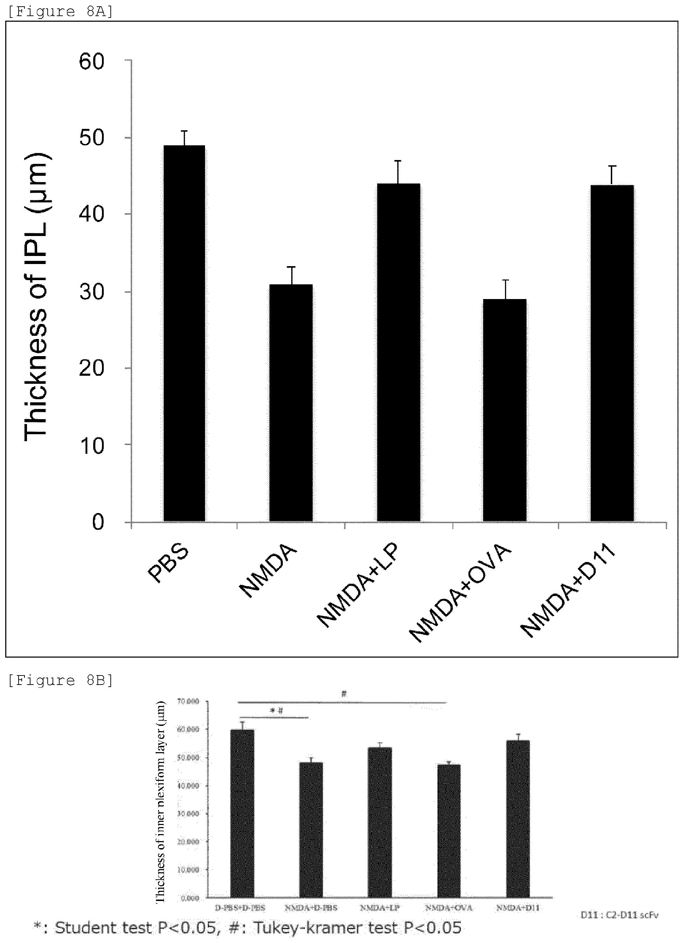

As a result of counting the number of cells in the ganglion cell layer (GCL) of HE stained images in Fig. 6 (4 to 6 cases), it was shown that scFv (C2-D11) has a protective effect on the decrease in the number of GCL cells by NMDA (Fig. 7). Further, as a result of measuring the thickness of the inner plexiform layer (IPL) in the HE stained images of Fig. 6 (4 to 6 cases), it was shown that scFv (C2-D11) has an inhibitory effect (protective effect) on the decrease in the thickness of IPL by NMDA (Fig. 8A). Further, Fig. 8B shows results obtained by requesting a third party organization to perform the same experiment. The results of Fig. 8B showed that good reproducibility was obtained.

### <Measurement of thickness of inner plexiform layer by optical coherence tomography>

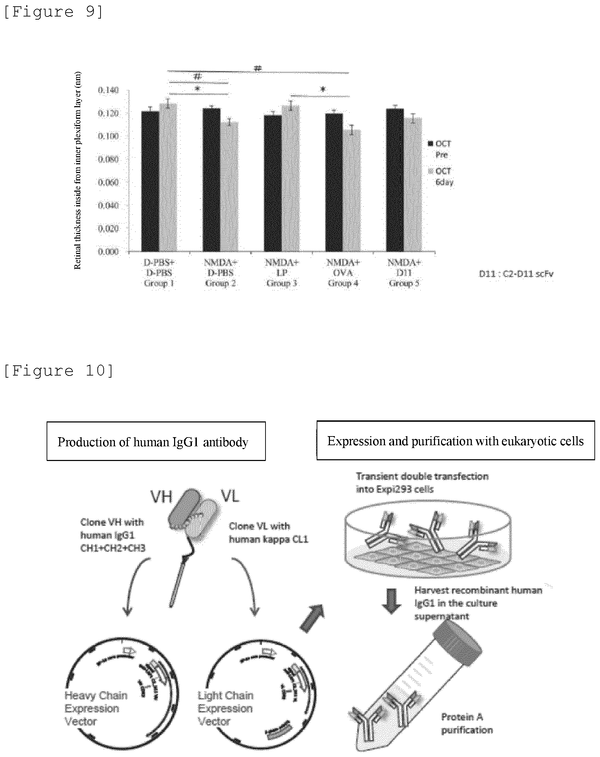

The thickness of the inner plexiform layer of the retina at the sixth day after treatment with each sample was measured by an optical coherence tomography (OCT). The thickness of the retinal inside from the inner plexiform layer is shown in Fig. 9. The thickness of the inner plexiform layer was significantly decreased in a NMDA administration group and a NMDA + OVA administration group compared to a PBS administration group, and no decrease was observed in a NMDA + LP administration group and a NMDA + C2-D11 scFv administration group. From these results, the retinal protective effect of C2-D11 scFv was confirmed.

### <Production of anti-LRP1 human IgG1 antibody from scFv antibody C2_D11>

As schematically shown in Fig. 10, the anti-LRP1 human IgG1 antibody was produced from C2_D11. From the scFv sequence of C2_D11, amino acid sequences corresponding to VL and VH, respectively, were analyzed from a gene sequence of a phagemid vector, and each of VL and VH was incorporated into a gene expression vector having a Fc region of human IgG1. Each plasmid was double-transfected into a eukaryotic cell expression system (HEK-293) to establish cells that transiently express the antibody. From the culture supernatant after culture for about one week, the expressed antibody was isolated and purified by affinity purification with protein A. pY03'-FLAG-C2-D11, a phagemid capable of expressing a fusion protein of the M13 phage with g3 protein, has been deposited under the accession number NITE BP-03322 in the National Institute of Technology and Evaluation, Biotechnology Center, Patent Microorganisms Depositary (NPMD) (Address: Room 122, Kazusa kamatari 2-5-8, Kisarazu-shi, Chiba) on November 20, 2020.

### <Binding specificity of anti-LRPl human IgGl antibody>

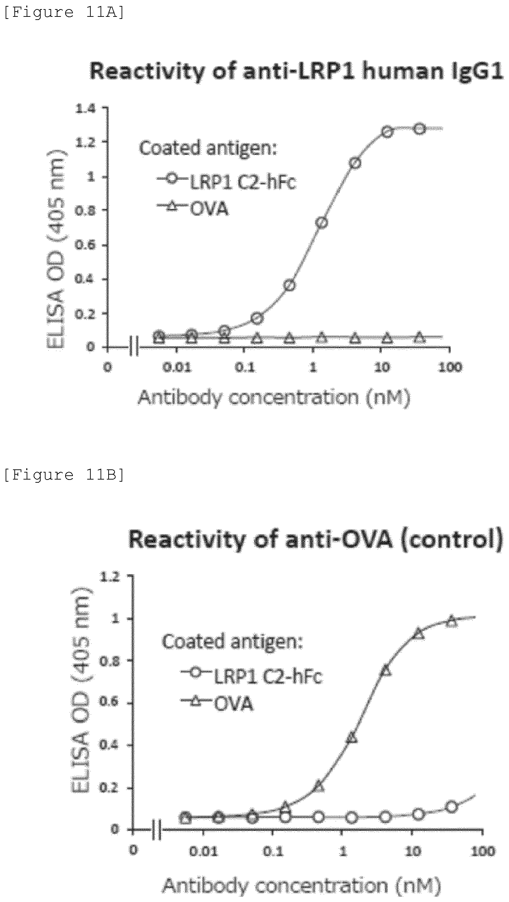

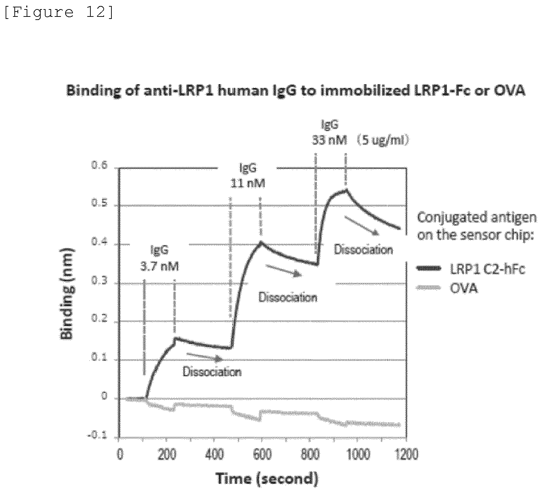

The binding specificity of an anti-LRPl human IgGl antibody to LRP1 was evaluated by ELISA and a biosensor. The results of ELISA for the anti-LRPl human IgGl antibody are shown in Fig. 11A, and the results of ELISA for an anti-OVA antibody as a control are shown in Fig. 11B. After immobilizing the C2 domain of LRP-1 to an ELISA plate, a purified antibody was added to the plate and the amount of bound antibody was quantified by colorimetric determination. Furthermore, the binding of the anti-LRPl human IgGl antibody to an immobilized LRP1-Fc was shown in Fig. 12. The affinity of the antibody for LRP-1 was determined by immobilizing the C2 domain protein of LRP-1 on a biosensor chip, continuously adding antibodies with C2_D11 in the VL and VH at the concentrations shown in Figure 12, and calculating the on-rate (Kon) and off-rate (Koff) values at each binding. From these results, a value of the dissociation constant (Kd) of the anti-LRPl human IgGl antibody prepared from C2_D11 was 0.9 nM, indicating that it has strong affinity and binding specificity for LRPl.

### <Intracellular calcium analysis in retinal ganglion cells (RGCs)>

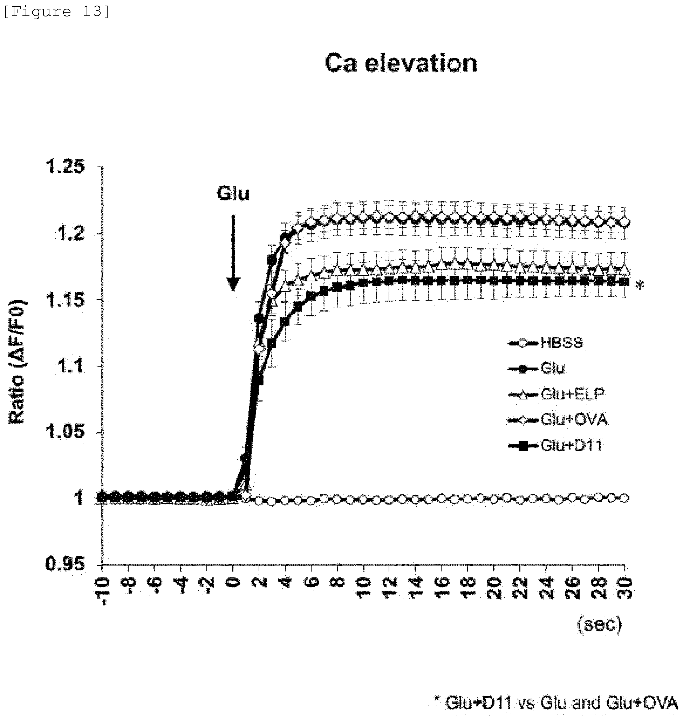

RGCs were maintained through an experiment with a heating element (Tokai Hit, Shizuoka, Japan). Cells were cultured in the microdish for at least 14 days and incubated for 30 minutes at 37°C with 3 µM Fluo-8 acetoxy metal ester (AAT Bioquest, Sunnyvale, CA). After washing the cells twice with 500 µl HBSS containing 2.4 mM CaCl₂, 20 mM HEPES, and no magnesium, 300 µM glutamic acid and 10 µM glycine with or without 100 ng/ml E-LP or 100 µg/ml C2-D11 IgG were administered. Fluorescence images were acquired every 500 msec using an ORCA-R2 digital CCD camera (Hamamatsu Photonics, Hamamatsu, Japan) and analyzed by MetaFluor fluorescence ratio imaging software (Molecular Devices, Sunnyvale, CA). In this test, 100 µg/ml C2-D11 IgG was evaluated using 100 ng/ml apolipoprotein E-containing lipoprotein (E-LP) as a positive control and the anti-OVA antibody as a negative control. The results are shown in Fig. 13.

Fig. 13 shows an evaluation of the protective effect on retinal ganglion cells using intracellular calcium influx by glutamate stimulation as an index. intracellular calcium influx was observed by 300 µM glutamic acid, and as a result, 100 µg/ml C2-D11 IgG exhibited a stronger inhibitory effect on intracellular calcium influx than E-LP. Since excessive calcium influx by 300 µM glutamic acid induces apoptosis, this result is considered to indicate one of neuroprotective mechanisms of C2-D11 IgG.

### <Frozen specimen preparation and immunofluorescence staining of rat eyeballs>

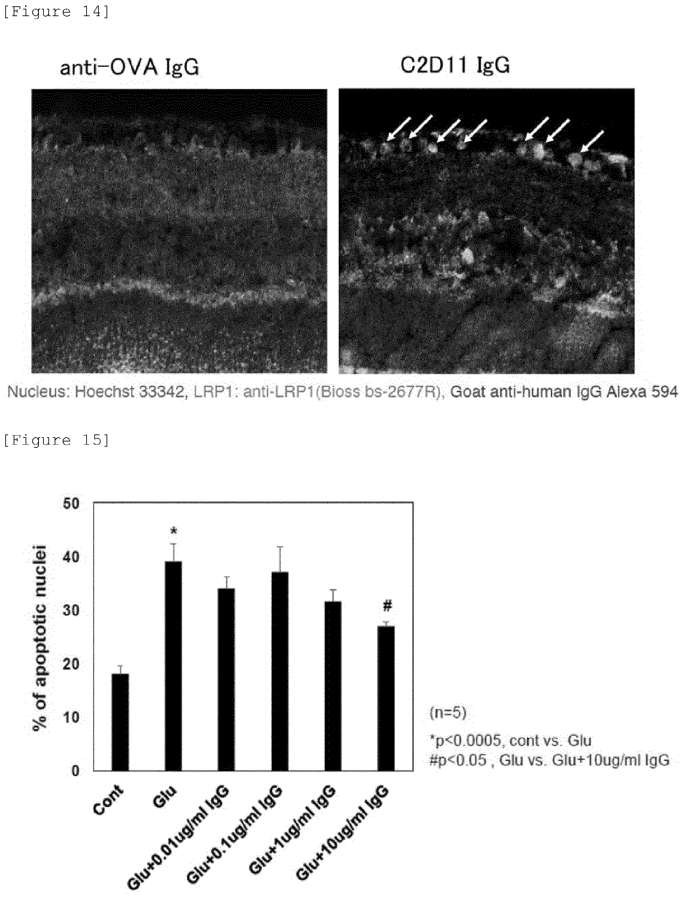

Frozen specimen preparation and immunofluorescence staining of rat eyeballs were performed in the following procedures.
(1) Thinly sliced sections were prepared in the same manner as in the HE staining method.
(2) 15% blocking buffer (in PBS, goat 15% serum, 0.05% Tween 20) was added and reacted at room temperature for one hour.
(3) Washing was performed with PBS for 15 minutes.
(4) A Primary antibody (human anti-LRP1 IgG in 1% blocking buffer) was added and incubated overnight at 4°C.
(5) Washing was performed with PBS for 15 minutes.
(6) A secondary antibody (Alexa Fluor 488-bound goat anti-rabbit IgG (dilution 1 : 500, Invitrogen)) and Alexa Fluor 594-bound goat anti-human IgG (dilution 1 : 500, Invitrogen)) were added and reacted at room temperature for one hour.
(7) Washing was performed with PBS containing Heochst33342 (1 : 2000 dilution) for one minute.
(8) After encapsulation, observation was performed with a fluorescence microscope. The results are shown in Fig. 14.

In the above experiment, it was observed by fluorescent immunostaining whether C2-D11 IgG binds to LRP1 of retinal ganglion cells after being administered into the vitreous body of rats. From the results shown in Fig. 14, it was revealed that C2-D11 IgG and LRP1 colocalize in the ganglion cell layer (arrows in the drawing). On the other hand, it was shown that the anti-OVA antibody was not colocalized. This result indicates that C2-D11 IgG binds to LRP1 on retinal ganglion cells and exerts a neuroprotective effect.

### <Neuroprotective effect of anti-LRP1 human IgG1 antibody on glutamate-induced retinal ganglion cell death>

Glutamic acid was administered in combination with various concentrations of the anti-LRP1 human IgG1 antibody, and the rate of cell death was examined. Specifically, 300 µM glutamic acid and 10 µM glycine were used to induce apoptosis for two hours. The anti-LRP1 human IgG1 antibody was used at a concentration of 0.01 ug/ml to 10 ug/ml. Nuclear aggregation 24 hours after glutamate-induced neurotoxicity was analyzed by Hoechst staining. The results are shown in Fig. 15. From Fig. 15, the neuroprotective effect was observed by an IgG antibody at 10 ug/mL (n = 5).

### <Production of recombinant protein of LRP1>

Various deficient variants of Cluster II of human low-density lipoprotein receptor-associated protein 1 (LRP1) were prepared for epitope mapping. A GST-tag was fused to each of the prepared deficient variants of Cluster II of human LRP1. A cDNA fragment of Cluster II of human low-density lipoprotein receptor-associated protein 1 (LRP1) was synthesized to encode forward linker SGGSTS, Cluster II (position, 786-1165) and backward linker ASTGS (Thermo Fisher Scientific, Waltham, MA). A cDNA fragment encoding each deficient variant of Cluster II was prepared by PCR using primer sets shown in the following table and the cDNA fragment of Cluster II as a template. An expression vector was obtained by digesting the cDNA fragment with BamHI and EcoRI and inserting it into the corresponding restriction site on pGEX-6P-1 expression vector (Cytiva, Marlborough, MA). BL21 cells having the expression vector were then cultured in MagicMedia (Thermo Fisher Scientific), and each recombinant protein (each deficient variant) was produced according to the manufacturer's protocol (Cytiva, Marlborough, MA). After lysis of E. coli by sonication, a GST fusion protein was pulled down with glutathione-sepharose (Cytiva) and used for immunoblotting.

### [Table 6]

**Table 6: Primer set**

| rProtein | Structure | Primer | ' Sequence (5'-3') |
|---|---|---|---|
| GST_LR PI. C2_dell | GST_EGF-like4_LDL receptor class A3_A4_A5_A6_A7 | LRP1_C2 _01 | GCGATGGATCCTCCGGAGGTTCCACTAGT (SEQ ID NO: 21) |
| | | LRP1_C2 _06 | GCATCAATTGTCAGGAACCAGTAGAAGCTCCCTCACAGCT CTTCTCATCGCT (SEQ ID NO: 22) |
| GST_LR P1-C2_del2 | GST EGF. like4_LDL receptor class A3_A4_A5 | LRP1_C2 _01 | GCGATGGATCCTCCGGAGGTTCCACTAGT (SEQ ID NO: 21) |
| | | LRP1_C2 _08 | GCATCAATTGTCAGGAACCAGTAGAAGCGGACTCGCAGTT CTCCTCGTCCGA (SEQ ID NO: 23) |
| GST_LR P1-C2 del3 | GST_EGF-like4_LDL receptor class A3_A4_A5_A6_A7_ A8_A9 | LRP1_C2 _01 | GCGATGGATCCTCCGGAGGTTCCACTAGT (SEQ ID NO: 21) |
| | | LRP1_C2 _02 | GCATCAATTGTCAGGAACCAGTAGAAGCGGCTGAACAAG TGGCATTGGACTC (SEQ ID NO: 24) |
| GST_LR P1-C2_del4 | GST_EGF-like4_LDL receptor class A3_A4_A5_A6_A7_ A8 | LRP1_C2 _01 | GCGATGGATCCTCCGGAGGTTCCACTAGT (SEQ ID NO: 21) |
| | | LRP1_C2 _04 | GCATCAATTGTCAGGAACCAGTAGAAGCGTTGGTGCAGTT GGCGTGTGTCTC (SEQ ID NO: 25) |

### <Identification of epitope>

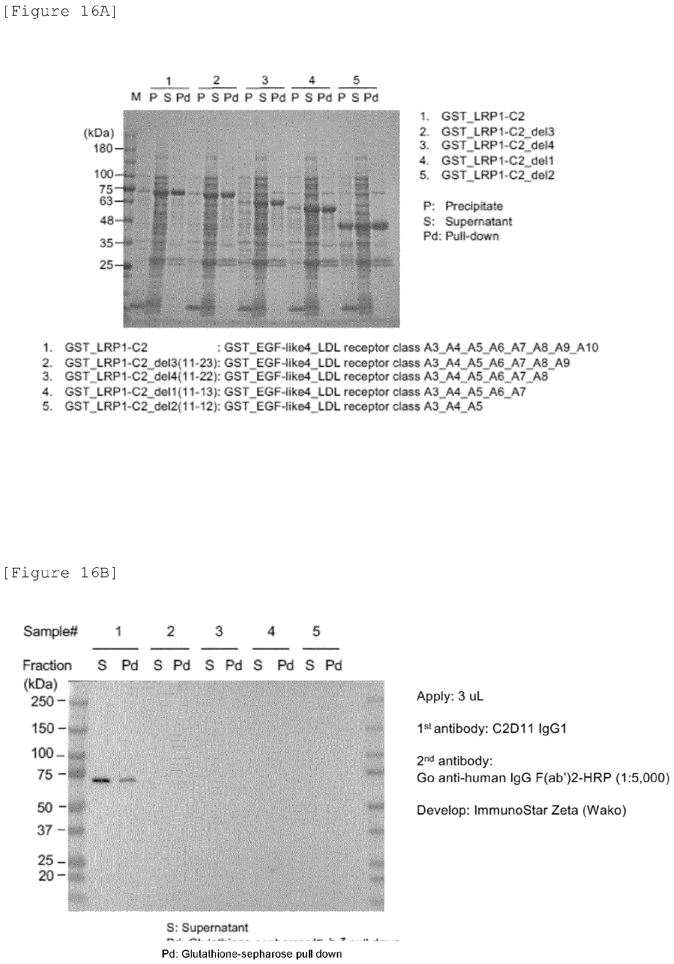

The prepared deficient variants were subjected to electrophoresis, and gel staining was performed using Coomassie Brilliant Blue R250. The results are shown in Fig. 16A. Further, immunoblotting of the prepared deficient variants was performed using C2-D11 IgG in 5% BSA/TBS-T in the same manner as shown in the section of <Immunoblotting> above. As a secondary antibody, a goat anti-human IgG bound to HRP was used. The results are shown in Fig. 16B. From Fig. 16B, an epitope of C2-D11 was identified.

## Claims

1. An optic nerve protecting agent comprising an anti-low density lipoprotein receptor-related protein 1 (LRP1) antibody or an anti-LRP1 antibody fragment.

2. The optic nerve protecting agent according to claim 1, wherein the anti-LRP1 antibody or the antibody fragment specifically binds to C2 domain of the LRP1.

3. The optic nerve protecting agent according to claim 1 or 2, wherein the anti-LRP1 antibody or the antibody fragment recognizes an epitope:
(a) in a region of amino acids at positions 786 to 1165 in an amino acid sequence of LRP1 having an amino acid sequence represented by SEQ ID NO: 1,
(b) in a region of amino acids at positions 787 to 1166 in an amino acid sequence of LRP1 having an amino acid sequence represented by SEQ ID NO: 2,
(c) in a region of amino acids at positions 786 to 1165 in an amino acid sequence of LRP1 having an amino acid sequence represented by SEQ ID NO: 3, or
(d) in a region of amino acids at positions 787 to 1166 in an amino acid sequence of LRP1 having an amino acid sequence represented by SEQ ID NO: 4.

4. The optic nerve protecting agent according to any one of claims 1 to 3, wherein
(i) the anti-LRP1 antibody or the antibody fragment comprises:
a heavy chain complementarity determining region 1 (VH CDR1) that comprises an amino acid sequence having a sequence identity of 80% or more to an amino acid sequence represented by SEQ ID NO: 9;
a heavy chain complementarity determining region 2 (VH CDR2) that comprises an amino acid sequence having a sequence identity of 80% or more to an amino acid sequence represented by SEQ ID NO: 10;
a heavy chain complementarity determining region 3 (VH CDR3) that comprises an amino acid sequence having a sequence identity of 80% or more to an amino acid sequence represented by SEQ ID NO: 11;
a light chain complementarity determining region 1 (VL CDR1) that comprises an amino acid sequence having a sequence identity of 80% or more to an amino acid sequence represented by SEQ ID NO: 12;
a light chain complementarity determining region 2 (VL CDR2) that comprises an amino acid sequence having a sequence identity of 80% or more to an amino acid sequence represented by SEQ ID NO: 13; and
a light chain complementarity determining region 3 (VL CDR3) that comprises an amino acid sequence having a sequence identity of 80% or more to an amino acid sequence represented by SEQ ID NO: 14,
or
(ii) the anti-LRP1 antibody or the antibody fragment comprises
a heavy chain variable region that comprises an amino acid sequence having a sequence identity of 80% or more to an amino acid sequence represented by SEQ ID NO: 15, and
a light chain variable region that comprises an amino acid sequence having a sequence identity of 80% or more to an amino acid sequence represented by SEQ ID NO: 16,
or
(iii) the anti-LRP1 antibody or the antibody fragment comprises an amino acid sequence having a sequence identity of 80% or more to an amino acid sequence represented by SEQ ID NO: 19.

5. The optic nerve protecting agent according to any one of claims 1 to 4, wherein the anti-LRP1 antibody or the antibody fragment is a humanized antibody or a human antibody.

6. The optic nerve protecting agent according to any one of claims 1 to 5, wherein the anti-LRP1 antibody or the antibody fragment binds to LRP1 with a dissociation constant (Kd) of 1 × 10⁻⁷ M to 1 × 10⁻¹¹ M.

7. A composition comprising the optic nerve protecting agent according to any one of claims 1 to 6.

8. The composition according to claim 7, wherein the composition is topically administered to a subject.

9. The composition according to claim 8, wherein the topical administration is intravitreal administration.

10. The composition according to claim 8 or 9, wherein the composition is in a form of eye drops.

11. The composition according to any one of claims 8 to 10, for treating glaucoma.

12. The composition according to any one of claims 8 to 11, wherein the subject has an intraocular pressure of 10 to 21 mmHg.

13. An anti-LRP1 antibody or an anti-LRP1 antibody fragment, comprising:
(i) a heavy chain complementarity determining region 1 (VH CDR1) that comprises an amino acid sequence having a sequence identity of 80% or more to an amino acid sequence represented by SEQ ID NO: 9;
a heavy chain complementarity determining region 2 (VH CDR2) that comprises an amino acid sequence having a sequence identity of 80% or more to an amino acid sequence represented by SEQ ID NO: 10;
a heavy chain complementarity determining region 3 (VH CDR3) that comprises an amino acid sequence having a sequence identity of 80% or more to an amino acid sequence represented by SEQ ID NO: 11;
a light chain complementarity determining region 1 (VL CDR1) that comprises an amino acid sequence having a sequence identity of 80% or more to an amino acid sequence represented by SEQ ID NO: 12;
a light chain complementarity determining region 2 (VL CDR2) that comprises an amino acid sequence having a sequence identity of 80% or more to an amino acid sequence represented by SEQ ID NO: 13; and
a light chain complementarity determining region 3 (VL CDR3) that comprises an amino acid sequence having a sequence identity of 80% or more to the amino acid sequence represented by SEQ ID NO: 14,
or
(ii) a heavy chain variable region that comprises an amino acid sequence having a sequence identity of 80% or more to an amino acid sequence represented by SEQ ID NO: 15, and
a light chain variable region that comprises an amino acid sequence having a sequence identity of 80% or more to an amino acid sequence represented by SEQ ID NO: 16,
or
(iii) an amino acid sequence having a sequence identity of 80% or more to an amino acid sequence represented by SEQ ID NO: 19.

14. A composition comprising the anti-LRP1 antibody or the anti-LRP1 antibody fragment according to claim 13.
